# EUROPEAN PATENT APPLICATION

(11) **EP 2 815 759 A1**
(43) Date of publication of application: **24.12.2014**
(21) Application number: 13749767.3
(22) Date of filing: 01.02.2013
(51) Int. Cl.: A61K 36/896, A61P 1/04, A61P 1/00

(54) **PHARMACEUTICAL COMPOSITION CONTAINING AS ACTIVE INGREDIENT EXTRACT FROM BARK OF LIRIODENDRON TULIPIFERA**

(30) Priority: 16.02.2012 KR 20120015766
(71) Applicant: Cho Dang Pharm. Co., Ltd., Seoul 152-050 (KR)
(72) Inventor: KIM, Nak Doo, Seoul 137-764 (KR)
(74) Representative: Harding, Charles Thomas
(86) International application number: PCT/KR2013/000820
(87) International publication number: WO 2013/122344

(57) **Abstract**

The present invention relates to a pharmaceutical composition containing, as active ingredients, epitulipinolide and costunolide extracted from the bark of Liriodendron tulipifera. More specifically, the present invention relates to a pharmaceutical composition for treating the gastrointestinal tract, the composition containing, as active ingredients, epitulipinolide and costunolide, which are the active ingredients of the extract from the bark of Liriodendron tulipifera, and to the use thereof as a therapeutic agent for gastrointestinal diseases.

## Description

This is a national phase application of PCT/KR2013/000820 filed on February 1, 2013, claiming priority to Korean Patent Application No.10-2012-0015766 filed on February 16, 2012, the contents of which are incorporated herein by references.

### Technical Field

The present invention relates to a pharmaceutical composition containing epitulipinolide and costunolide extracted from bark of Liriodendron tulipifera as active ingredient. More particularly, the present invention relates to a pharmaceutical composition and/or a therapeutic agent for treating the gastrointestinal disease containing epitulipinolide and costunolide extracted from bark of Liriodendron tulipifera as active ingredient.

### Description of Prior Art

It has been reported that the gastritis and/or stomach ulcer is the most common disease, because about 10% of Korean populations have suffered from gastritis and/or stomach ulcer more than once in a whole life. The causes of gastritis and/or stomach ulcer have been known by the digestion of stomach wall containing mucosa, sub-mucosa, muscularis externa and serosa by gastric acid. Gastritis has been referred if only gastric mucosa has been damaged, while stomach ulcer has been referred if gastric sub-mucosa and muscularis externa have been damaged. Further, the unbalance between offensive factor and defensive factor may cause the generation of gastritis and/or stomach ulcer, for example, the increase of offensive factor or the decrease of defensive factor.

The increase of secretion of gastric acid and/or pepsin can be an example of increase of offensive factor, while the defect of gastric mucosa, the decrease of mucus secretion, the decrease of bicarbonate ion secretion and/or the decrease of prostaglandin production can be an example of decrease of defensive factor. On the other hand, it has been known that the infection of Helicobacter pylori can cause the gastritis and/or stomach ulcer.

Gastric acid hyper-secretion has been regarded as the main cause of gastritis and/or stomach ulcer. The gastric acid, which is hydrochloric acid secreted from stomach wall cells has a role for digesting proteins by activating pepsin as well as damaging the stomach wall. The therapeutic agent for treating gastritis and/or stomach ulcer can be classified into the drug inhibiting the offensive factor, for example, gastric acid, pepsin, smoke, oxygen free radical and/or alcohol, and the drug enhancing the defensive factor.

As an offensive factor inhibitor, antacid and/or gastric acid secretion inhibitor can be exemplified. Further, as a gastric acid secretion inhibitor, the antagonist of H2 receptor, for example, cimetidine, ranitidine and/or famotidine etc.; the proton pump inhibitor (PPI), for example, omeprazole and/or lansoprazole can be exemplified. Especially, almost 100% of duodenal ulcer and more than 90% of stomach ulcer can be initially recovered by administration of PPI drug. However, in spite of improving initial recovery rate of duodenal ulcer and/or stomach ulcer, the recurrence rate has not been reduced.

On the other hand, a defensive factor enhancer can induce the covering of the ulcerative lesion, the synthesis and secretion of mucus, the increase of blood flow in gastric mucosa, the increase of endogenous prostaglandin and/or the increase of tissue regeneration. Further, it has been known that a defensive factor enhancer has been useful for preventing the recurrence of duodenal ulcer and/or stomach ulcer. Of course, the sterile therapy of Helicobacter pylori also can be considered as important treating method against ulcer.

However, the causes of gastritis and/or stomach ulcer have not been clearly disclosed since complex causes result in gastritis and/or stomach ulcer. Therefore, the absolute treating method has not been developed.

On the other hand, Liriodendron tulipifera L. is a kind of deciduous broad-leaved arboreal belonged to Magnoliaceae family. It has been used as a raw material of pulp. In the bark, leave and/or wood of Liriodendron tulipifera, alkaloid, sesquiterpene and/or lignan has been known to be included.

It has been known that aporphine alkaloid having anti-bacterial activity has been contained in the bark of Liriodendron tulipifera (Hufford C. D. and Funderburk M. J., J. Pharm. Sci. 63: pp.1338-1339, 1974; Hufford C. D. et al., J. Pharm. Sci. 64: pp.789-792, 1975). Further, the bark extract containing this alkaloid has been used for treating malaria since Unites States War of Independence. On the other hand, glaucine alkaloid has been used for antitussive agent in Soviet Union.

According to the research in 1975, the alkaloid fraction extracted from the bark of Liriodendron tulipifera has been reported as having anti-bacterial activity. Further, it has been reported that N-methyllaurotetanine, lirioferine and/or liriotulipiferine has an anti-fungal activity (Huang Hsu, C-Y, 1976. MS thesis, North Carlolina State University, Raleigh). Further, it has been also reported that dehydroglaucine and/or liriodenine has cytotoxicity and anti-bacterial activity (Warthen D, Gooden E L and Jacobson M., J. Pharm. Sci., 58: pp.637-638(1969); Hufford C D, Funderburk M J, Morgan J M. et al., J. Pharm. Sci., 64: pp.789-792(1975); Chen C R, Bed J L, Doskotch R W et al., Llovdia 37: pp.493-500(1974)).

On the other hand, it has been also disclosed that sesquiterpene lactone extracted from bark of Liriodendron tulipifera has an anti-tumor activity. Further, costunolide, tulipinolide, epi-tulipinolide, epi-tulipdienolide and/or gamma-liriodenolide has been also disclosed as an effective ingredient of the bark of Liriodendron tulipifera (Doskotch R. W and EL-Feraly F. S., J Pharm. Sci. 58: pp.877-880, 1969; Doskotch R. W and E LI-Feraly F. S., J. Org. Chem. 35: pp.1928-1936, 1970; Moon M. K. et al., Arch. Pharm .Res. 30: pp.299-302, 2007).

Further, in the leaf of Liriodendron tulipifera , lipiferolide, epitulipinolide diepoxide and/or peroxyferolide has been also disclosed as an effective ingredient (Doskoch R W, EL-Feraly F S, Fairchild E H, J. Chem. Soc (Chem. Comm) pp.402-403, 1976; Doskotch R W, EL-Feraly F S, Fairchild E H, J. Org. Chem. 42: pp.3614-3618, 1977). On the other hand, in the bark of Liriodendron tulipifera , lignan derivatives, pinoresinol, (+)-syringaresinol and/or liriodendrin in the form of monoglycoside or diglycoside has been also disclosed as an effective ingredient (Dickey E E., J. Org. Chem. 23: pp.179-184, 1958; Fujimoto H and Higuchi T., J. Jap. Wood Res. Soc, 23: pp.405-410, 1977). Further, it has been reported that liriodendrin causes the bone growth in animal (Korean patent registration No. 10-597,563).

The inventor of present application has found that extract from the bark of Liriodendron tulipifera containing epitulipinolide and costunolide as active ingredients can be applied for treating gastritis and/or stomach ulcer. Further, various kinds of small amount materials have been isolated in the extract composition from the bark of Liriodendron tulipifera.

Therefore, the inventor of present application has prepared a pharmaceutical composition containing epitulipinolide and costunolide as active ingredients from the bark of Liriodendron tulipifera for treating gastritis and/or stomach ulcer. Finally, the present invention has been completed by developing a pharmaceutical composition containing epitulipinolide and costunolide as active ingredients from the bark of Liriodendron tulipifera for treating gastritis and/or stomach ulcer.

### Problem to be solved

The problem to be solved is to develop a pharmaceutical composition containing epitulipinolide and costunolide as active ingredients from the bark of Liriodendron tulipifera for treating gastritis and/or stomach ulcer. Accordingly, the development of pharmaceutical composition containing epitulipinolide and costunolide as active ingredients from the bark of Liriodendron tulipifera has been carried out for treating gastritis and/or stomach ulcer.

### Means for solving the problem

The object of present application is to provide a pharmaceutical composition from the bark of Liriodendron tulipifera comprising 0.01∼50 wt% of epitulipinolide and costunolide as active ingredients for preventing or treating gastritis and/or stomach ulcer.

Further, said pharmaceutical composition further comprises epitulipdienolide, ridentin and/or deacetyllipiferolide as another active ingredient.

Further, said pharmaceutical composition further comprises the pharmaceutically acceptable carrier.

The another object of present application is to provide an oral preparation comprising said pharmaceutical composition, wherein said preparation is at least one selected from of tablet, pill, powder, hard capsule, gelatin banding hard capsule, soft capsule, chewing tablet in the form of caramel or jelly and/or oral solution.

Further, said oral preparation is selected from soft capsule or oral solution.

### Advantageous effect

The outstanding advantageous effect of present application is to provide a pharmaceutical composition containing epitulipinolide and costunolide as active ingredients for treating gastritis and/or stomach ulcer. Accordingly, the development of pharmaceutical composition containing epitulipinolide and costunolide as active ingredients has been made for treating gastritis and/or stomach ulcer.

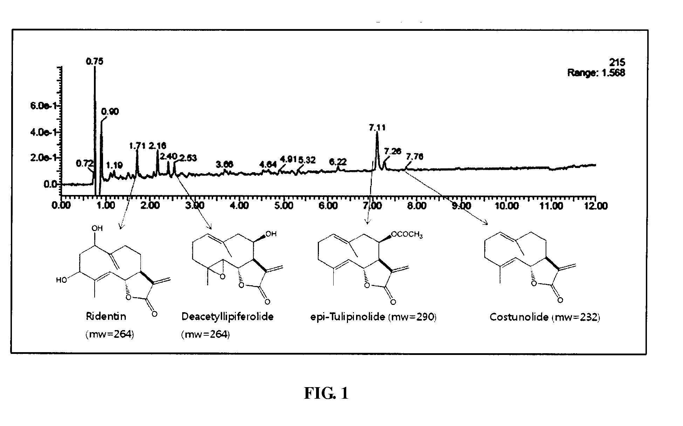

On the other hand, the pharmaceutical composition containing epitulipinolide and costunolide as active ingredients has an efficacy for preventing or treating gastrointestinal disease. Especially, the pharmaceutical composition has an excellent efficacy for treating gastritis and/or stomach ulcer. Further, oral preparation of this pharmaceutical composition can make an outstanding increase of bioavailability by increasing solubility and dissolution rate of insoluble components. Fig 1

### Brief description of drawings

Fig. 1 shows LC-MS chromatography data of isolated extract from the bark of Liriodendron tulipifera according to Preparation Example 1. In the extract of Preparation Example 1, ridentin (Mw=264), deacetyllipiferolide (Mw=264), epitulipinolide (Mw=290) and/or costunolide (Mw=232) has been confirmed to be included.

### Preferred embodiment of invention

The present invention is to provide a pharmaceutical composition and/or an oral preparation for treating the gastrointestinal disease containing epitulipinolide and costunolide extracted from bark of Liriodendron tulipifera as active ingredient.

The extract from the bark of Liriodendron tulipifera has been prepared by the extraction solvent selected from ethyl acetate, dichloromethane, alcohol, alcohol aqueous solution and/or mixture of them. Further, epitulipinolide and costunolide has been included in this extract. Of course, epitulipinolide and costunolide has been used as component indicator of the extract from the bark of Liriodendron tulipifera.

Of course, the extract from the bark of Liriodendron tulipifera has an excellent efficacy for treating gastrointestinal disease. For measuring the efficacy for treating gastritis and/or stomach ulcer, various kinds of in vivo animal test models have been employed. For example, water-immersion-restraint stress model, HCl-EtOH inducing gastritis model and/or indomethacin inducing stomach ulcer model has been employed. As a result of test, the extract from the bark of Liriodendron tulipifera shows an excellent efficacy to the gastritis and/or stomach ulcer by promoting the prostaglandin biosynthesis.

On the other hand, the present invention provide a pharmaceutical composition from the bark of Liriodendron tulipifera comprising epitulipinolide and costunolide as active ingredients for preventing or treating gastrointestinal disease.

Further, said pharmaceutical composition further comprises epitulipdienolide, ridentin and/or deacetyllipiferolide as another active ingredient.

The pharmaceutical composition of present invention can be administered in various routes, for example, oral, intravenous and/or intramuscular. The dose of pharmaceutical composition is 10∼500mg per one day.

Preferred formulation of pharmaceutical composition of present invention is an oral preparation which is at least one formulation selected from of tablet, pill, powder, hard capsule, gelatin banding hard capsule, soft capsule, chewing tablet in the form of caramel or jelly and/or oral solution. Further, soft capsule or oral solution is the most desirous.

The pharmaceutical composition of present invention can include pharmaceutically acceptable carriers. For example, the additive, such as, acid, fatty acid and/or fatty alcohol can be included to improve the solubility of extract from bark of Liriodendron tulipifera as well as to improve the dissolution rate of extract from bark of Liriodendron tulipifera by increasing its dispersion. Further, the sugar component, such as, white sugar, maltose, purified white sugar, starch syrup can be included. Of course, any inorganic additive, such as, magnesium stearate, talc as well as diluent, such as, non-crystalline cellulose, calcium hydrogen phosphate, starch and/or mannitol can be included. Further, anti-oxidant, flavoring agent, preservative, flavor, sweetener, pigment, pH adjusting agent and/or viscosity adjusting agent can be included. The amount of this additive has been conventionally known.

As the acid, the fatty acid or the fatty alcohol in the pharmaceutical composition of present invention, citric acid, oleic acid, stearyl alcohol, myristic acid, linoleic acid, lauric acid, capric acid, caprylic acid and/or caproic acid can be used, but not be limited thereto.

As the antioxidant in the pharmaceutical composition of present invention, butylated hydroxy toluene, sodium bisulfite, α-tocopherol, vitamin C, β-carotene, tocopherol acetate, fumarate acid, nalic acid, butylated hydroxyanisole, propyl gallate and/or sodium ascorbate can be used, but not be limited thereto.

As the flavoring agent in the pharmaceutical composition of present invention, mixed fruit flavor, apple flavor, strawberry flavor, cherry flavor, mint, vanilla flavor, yogurt flavor, or drink flavor can be used, but not be limited thereto.

As the preservative in the pharmaceutical composition of present invention, benzoic acid, sodium benzoate, ethylparaben, methylparaben or propylparaben can be used, but not be limited thereto.

As the flavor in the pharmaceutical composition of present invention, menthol, peppermint oil, orange oil, clove oil, cinnamon oil, strawberry essence and/or other conventional plant extract or fruit aroma can be used, but not be limited thereto.

As the sweetener in the pharmaceutical composition of present invention, refined sugar, glucose, fructose, aspartame, stevioside, sorbitol, mannitol, oligosaccharide and/or starch syrup can be used, but not be limited thereto.

As the pigment in the pharmaceutical composition of present invention, Green No. 3, Red No. 2, Red No. 3, Blue No. 1, Blue No. 2, Yellow No. 4, Yellow No. 5, soluble mannitol, caramel, titanium oxide or ferric oxide can be used, but not be limited thereto.

As the pH adjusting agent in the pharmaceutical composition of present invention, sodium carbonate, sodium hydroxide, potassium hydroxide, triethanolamine or monoethanolamine can be used, but not be limited thereto.

As the viscosity adjusting agent in the pharmaceutical composition of present invention, hydroxypropyl cellulose (HPC), hydroxypropylmethylcellulose (HPMC), hydroxyethyl cellulose, ethyl cellulose, methyl cellulose, carboxymethyl cellulose, acacia, bentonite, alginic acid, propylene glycol alginate, polyvinylpyrrolidone, polyvinyl alcohol, carbopol, polycarbopil, tragacanth or xanthan can be used, but not be limited thereto.

The preparation method of extract from the bark of Liriodendron tulipifera can be explained as follows.

The preparation method of present application comprises 2 preparation steps; (1) preparation step of extract from the bark of Liriodendron tulipifera using specific solvent of ethyl acetate or dichloromethane (Method 1), (2) purification step for obtaining purified material enhancing the amount of component indicator from the extract obtained in step 1(Method 2).

In Method 1, after chopping and powdering the bark of Liriodendron tulipifera, the extraction has been carried out using extraction solvent selected from ethyl acetate or dichloromethane. After concentrating extract, the obtained material has contained epitulipinolide and costunolide as active ingredient.

In Method 2, the extract obtained in Method 1 has been dissolved using alcohol aqueous solution. After adding hexane, the lipid ingredient dissolved in hexane as well as water insoluble material have been removed. After separating alcohol aqueous solution layer, high purity of epitulipinolide and costunolide has been obtained. Further, said alcohol aqueous solution can be diluted into low concentration of alcohol aqueous solution by adding water. After adding dichloromethane to the obtained alcohol aqueous solution, epitulipinolide and costunolide can be isolated and purified in the dichloromethane fraction.

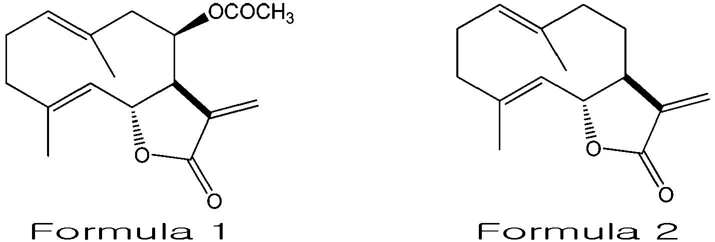

Obtained fraction according to Method 1 and Method 2 has been concentrated and dried. Finally, the extract composition can be made after removing remained solvent in the fraction

Therefore, the present invention provides a method for extracting and purifying epitulipinolide (formula 1) and costunolide (formula 2) from the bark of Liriodendron tulipifera.

The bark of Liriodendron tulipifera has been collected from the Liriodendron tulipifera located Jeollanam-do, gangjingun in Republic of Korea. After chopping and powdering the collected bark of Liriodendron tulipifera, extraction solvent has been added in a 2∼20 times amount, preferably 4∼10 times amount of collected bark. The extraction has been carried out for 24∼96 hours at 15∼50□. Finally, the extract has been obtained after concentrating and drying it at reduced pressure.

The extract obtained from the bark of Liriodendron tulipifera has been analyzed for measuring epitulipinolide and costunolide amount using Agilent HPLC 1200 series (U.S.A.) according to high performance liquid chromatography method. The conditions for HPLC analysis are as follows. Reverse phase column Kromasil C18, 5mg/ml of sample concentration, 215nm of UV wave length (flow rate: 1.0ml/min), mobile phase gradient condition has been shown in Table 1.

**[Table 1] Mobile phase gradient condition for HPLC analysis**

| retention time(min.) | water (wt%) | methanol (wt%) |
|---|---|---|
| 0 | 98 | 2 |
| 10 | 50 | 50 |
| 60 | 0 | 100 |
| 70 | 0 | 100 |
| 80 | 98 | 2 |

As a result of HPLC analysis according to gradient condition as shown in Table 1, the main components of epitulipinolide and costunolide have been detected at 31 minute and at 36 minute of retention time respectively. Further, to confirm the presence of epitulipinolide and costunolide respectively, each retention time of the standard of epitulipinolide and the standard of costunolide has been measured as the same manner. Upon comparing the retention time between standard and obtained material, we have found that epitulipinolide and costunolide have been isolated and measured.

In the aspect of stereochemistry, the specific rotation of epitulipinolide has been measured. The measured value of epitulipinolide ([α]D=+74) from the extract of Liriodendron tulipifera has almost corresponded with reported value of epitulipinolide ([α]D=+76). Therefore, the presence of epitulipinolide has been confirmed by stereochemistry.

On the other hand, each molecular weight of epitulipinolide, costunolide and other components from the extract of Liriodendron tulipifera has been measured according to LC-MS. It has been confirmed that the molecular weight of epitulipinolide is 290, while the molecular weight of costunolide is 232. Further, other components, ridentin (Mw=264) and deacetyllipiferolide (Mw=264) have been also measured and confirmed.

LC-MS analysis instrument made by Waters has been employed using reverse phase column. 2µl of sample volume concentration, 200∼500nm of UV wave length (flow rate: 0.3ml/min), mobile phase gradient condition has been shown in Table 2.

**[Table 2] Mobile phase gradient condition for LC-MS analysis**

| retention time(min.) | water (wt%) | ACN (wt%) |
|---|---|---|
| 0 | 85 | 15 |
| 10 | 30 | 70 |
| 60 | 0 | 100 |
| 70 | 0 | 100 |

The present application can be explained more concretely by following Preparation Examples and Examples. However, the scope of present application cannot be limited by following Examples.

### (Preparation Example 1) Preparation of isolated and purified extract from bark of Liriodendron tulipifera

200g of dried and chopped bark of Liriodendron tulipifera has been extracted using 800ml of ethyl acetate at 50 °C for 24 hours. Obtained extracted mixture has been concentrated and dried at reduced pressure. Finally, 6.83g of extract has been obtained. The amount of epitulipinolide has been 3.96 wt% and the amount of costunolide has been 0.18 wt%.

1g of extract obtained in above step has been dissolved with 50ml of 70% ethanol. After adding 50ml of hexane, the mixture has been agitated and fractioned. Obtained 70% ethanol fraction has been isolated and dried. Finally, 0.483g of purified extract has been obtained. The amount of epitulipinolide has been 9.72 wt% and the amount of costunolide has been 0.28 wt%.

Fig. 1 shows LC-MS chromatography data of isolated extract from the bark of Liriodendron tulipifera according to Preparation Example 1. In the extract of Preparation Example 1, ridentin (Mw=264), deacetyllipiferolide (Mw=264), epitulipinolide (Mw=290) and/or costunolide (Mw=232) has been confirmed to be included.

### (Preparation Example 2) Preparation of isolated and purified extract from bark of Liriodendron tulipifera

200g of dried and chopped bark of Liriodendron tulipifera has been extracted using 800ml of ethyl acetate at 50 °C for 48 hours. Obtained extracted mixture has been concentrated and dried at reduced pressure. Finally, 7.67g of extract has been obtained. The amount of epitulipinolide has been 3.94 wt% and the amount of costunolide has been 0.14 wt%.

1g of extract obtained in above step has been dissolved with 50ml of 70% ethanol. After adding 50ml of hexane, the mixture has been agitated and fractioned. Obtained 70% ethanol fraction has been isolated and dried. Finally, 0.571g of purified extract has been obtained. The amount of epitulipinolide has been 10.94 wt% and the amount of costunolide has been 0.30 wt%.

### (Preparation Example 3) Preparation of isolated and purified extract from bark of Liriodendron tulipifera

200g of dried and chopped bark of Liriodendron tulipifera has been extracted using 800ml of dichloromethane at 36 °C for 72 hours. Obtained extracted mixture has been concentrated and dried at reduced pressure. Finally, 4.95g of extract has been obtained. The amount of epitulipinolide has been 5.10 wt% and the amount of costunolide has been 0.35 wt%.

1g of extract obtained in above step has been dissolved with 50ml of 70% ethanol. After adding 50ml of hexane, the mixture has been agitated and fractioned. After obtaining ethanol fraction, 40ml of water and 90ml of dichloromethane have been added and agitated. Obtained dichloromethane fraction has been isolated and dried. Finally, 0.071g of purified extract has been obtained. The amount of epitulipinolide has been 23.43 wt% and the amount of costunolide has been 0.79 wt%.

### (Preparation Example 4) Preparation of isolated and purified extract from bark of Liriodendron tulipifera

200g of dried and chopped bark of Liriodendron tulipifera has been extracted using 800ml of dichloromethane at 36 °C for 24 hours. Obtained extracted mixture has been concentrated and dried at reduced pressure. Finally, 9.12g of extract has been obtained. The amount of epitulipinolide has been 9.11wt% and the amount of costunolide has been 0.29 wt%.

1g of extract obtained in above step has been dissolved with 50ml of 70% ethanol. After adding 50ml of hexane, the mixture has been agitated and fractioned. After obtaining ethanol fraction, 40ml of water and 90ml of dichloromethane have been added and agitated. Obtained dichloromethane fraction has been isolated and dried. Finally, 0.069g of purified extract has been obtained. The amount of epitulipinolide has been 21.19 wt% and the amount of costunolide has been 0.94 wt%.

In the extract of Preparation Examples, epitulipdienolide (Mw=290), ridentin (Mw=264), deacetyllipiferolide (Mw=264) can be additionally included.

The in vivo animal test results regarding the extract of bark of Liriodendron tulipifera can be explained as follows. For measuring the efficacy for treating gastritis and/or stomach ulcer, various kinds of in vivo animal test models have been employed. In other words, HCl-EtOH inducing gastritis model, water-immersion-restraint stress model and indomethacin inducing stomach ulcer model have been employed.

### (Example 1) Efficacy of extract from bark of Liriodendron tulipifera according to HCl-EtOH inducing stomach ulcer model

After white rats have been fasted for 24 hours, the extract of bark of Liriodendron tulipifera obtained in Preparation Example 1 has been orally administered according to method disclosed in Mizui et al., Jap. J. Pharmacol. 33: pp.939-945, 1983. After one hour, 1.5ml of 60% ethanol including 150mM HCl has been orally administered to each animal. One hour later, animals have been sacrificed by cervical dislocation. After removing the stomach, the stomach has been fixed by injection of 13ml of 2% formalin for 1 hour. After fixed stomach has been spread out, the lesion area has been measured using the photo from digital camera. Computer program Image J 1.38 (NIH, Bethesda, MD) has been applied for measuring gastric lesion area (mm²). The lesion inhibition rate has been calculated according to following equation. Further, 50% effective dose (ED50) has been also calculated using regression analysis.

Lesion inhibition rate(%)= {gastric lesion area of control group(mm²)- gastric lesion area of drug administration group(mm²)}/gastric lesion area of control group(mm²)×100

Efficacy of extract from bark of Liriodendron tulipifera obtained in Preparation Example 1 according to HCl-EtOH inducing stomach ulcer model has been shown in Table 3. The gastric lesion area of control group has been 233.4±30.9mm². Efficacy has been measured in 1mg/kg, 3mg/kg, 10mg/kg, 30mg/kg administration groups. The lesion inhibition rate of 30mg/kg administration group has been calculated into 92.0%.

**[Table 3] Efficacy of extract from bark of Liriodendron tulipifera according to HCl-EtOH inducing stomach ulcer model**

| test group | dose(mg/kg) | number of animals | gastric lesion area(mm²) | lesion inhibition rate(%) |
|---|---|---|---|---|
| control | - | 7 | 233.4±30.9 | - |
| extract from bark of L. tulipifera | 1 | 7 | 155.6±10.9* | 33.3 |
| | 3 | 7 | 125.4±33.2* | 46.3 |
| | 10 | 7 | 76.4±15.9* | 67.3 |
| | 30 | 7 | 18.8±4.8* | 92.0 |

| | | | | |
|---|---|---|---|---|
| *: Significant difference compared to the control group (p<0.05) | | | | |

### (Example 2) Efficacy of extract from bark of Liriodendron tulipifera according to water-immersion-restraint stress model

After white rats have been fasted for 24 hours, the extract of bark of Liriodendron tulipifera obtained in Preparation Example 1 has been orally administered according to method disclosed in Takagi et al., Jap. J. Pharmacol. 18: pp.9-18, 1968. 30 minutes later, white rats have been fixed in stress cage. The stress cage has been laid on 21±1°C of water for 8 hours until xiphoid of rats has been immersed. 8 hour later, animals have been sacrificed by cervical dislocation. After removing the stomach of rat, the stomach has been fixed by injection of 13ml of 2% formalin for 1 hour. After fixed stomach has been spread out, the lesion area has been measured using the photo from digital camera. Computer program Image J 1.38 (NIH, Bethesda, MD) has been applied for measuring gastric lesion area (mm²). The lesion inhibition rate and 50% effective dose (ED50) have been calculated as the same manner of HCl-EtOH model.

**[Table 4] Efficacy of extract from bark of Liriodendron tulipifera according to water-immersion-restraint stress model**

| test group | dose(mg/kg) | number of animals | gastric lesion area(mm²) | lesion inhibition rate(%) |
|---|---|---|---|---|
| control | - | 6 | 20.1±1.8 | - |
| extract from bark of L. tulipifera | 1 | 6 | 16.8±3.7 | 16.3 |
| | 3 | 6 | 11.1±2.3* | 45.0 |
| | 10 | 6 | 6.7±2.1* | 66.7 |
| | 30 | 6 | 3.5±1.0* | 82.4 |

| | | | | |
|---|---|---|---|---|
| *: Significant difference compared to the control group (p<0.05) | | | | |

Efficacy of extract from bark of Liriodendron tulipifera obtained in Preparation Example 1 according to water-immersion-restraint stress model has been shown in Table 4. The gastric lesion area of control group has been 20.1±1.8mm². The lesion inhibition rates have been measured into 16.3%, 45.0%, 66.7%, 82.4% respectively with respect to 1mg/kg, 3mg/kg, 10mg/kg, 30mg/kg administration groups. Therefore, the lesion area has been reduced regarding administration groups in a dose dependent manner.

### (Example 3) Efficacy of extract from bark of Liriodendron tulipifera according to indomethacin inducing stomach ulcer model

After 250g weight of male white rats have been fasted for 24 hours, the extract of bark of Liriodendron tulipifera obtained in Preparation Example 1 has been orally administered according to method disclosed in Yamasaki et al., Jap. J. Pharmacol. 49: pp.441-448, 1989. After one hour, indomethacin (50mg/kg) has been orally administered to each animal. After fasting for 5 hours, animals have been sacrificed by cervical dislocation. After removing the stomach, the stomach has been fixed by injection of 13ml of 2% formalin for 1 hour. After fixed stomach has been spread out, the lesion area has been measured using the photo from digital camera. Computer program Image J 1.38 (NIH, Bethesda, MD) has been applied for measuring gastric lesion area (mm²). The lesion inhibition rate and 50% effective dose (ED50) have been calculated as the same manner of HCl-EtOH model.

**[Table 5] Efficacy of extract from bark of Liriodendron tulipifera according to indomethacin inducing stomach ulcer model**

| test group | dose(mg/kg) | number of animals | gastric lesion area(mm²) | lesion inhibition rate(%) |
|---|---|---|---|---|
| control | - | 7 | 82.5±5.3 | - |
| extract from bark of L. tulipifera | 1 | 6 | 67.3±6.5* | 18.5 |
| | 3 | 6 | 51.4±7.4* | 37.8 |
| | 10 | 7 | 32.8±5.4* | 60.2 |
| | 30 | 7 | 19.0±3.7* | 77.0 |

| | | | | |
|---|---|---|---|---|
| *: Significant difference compared to the control group (p<0.05) | | | | |

Efficacy of extract from bark of Liriodendron tulipifera obtained in Preparation Example 1 according to indomethacin inducing stomach ulcer model has been shown in Table 5. The gastric lesion area of control group has been 82.5±5.3mm². The lesion inhibition rates have been measured into 18.5%, 37.8%, 60.2%, 77.0% respectively with respect to 1mg/kg, 3mg/kg, 10mg/kg, 30mg/kg administration groups. Therefore, the lesion area has been reduced regarding administration groups in a dose dependent manner.

### (Example 4) Efficacy of extract from bark of Liriodendron tulipifera according to acetic acid inducing stomach ulcer model

Efficacy of extract from bark of Liriodendron tulipifera obtained in Preparation Example 1 has been measured according to acetic acid inducing stomach ulcer model. Only solvent has been administered into control group, while extract from the bark of Liriodendron tulipifera has been administered into experimental group. Further, ranitidine and rebamipide used for drug for treating stomach ulcer have been also administered into experimental group.

The experiment has been carried out according to the method disclosed in Okabe and Pfeifferi, Did. Dis. 17, pp.619-628, 1972. 250g weights of male white rats have been anesthetized with ether and the stomach has been exposed. 50µl of 20% acetic acid has been injected into the subserosa in fundus of stomach using microliter syringe. Then, the abdomen of rat has been sutured. The drug has been orally administered into the rats at 10:00 am once a day for 10 days after operation. After 4 hour from final administration, animals have been sacrificed by ether. After removing the stomach of rat, the stomach has been spread out. The lesion area has been measured using the photo from digital camera. Computer program Image J 1.38 (NIH, Bethesda, MD) has been applied for measuring gastric lesion area (mm²). The lesion inhibition rate and 50% effective dose (ED50) have been calculated as the same manner of HCl-EtOH model.

**[Table 6] Efficacy of extract from bark of Liriodendron tulipifera according to acetic acid inducing stomach ulcer model**

| test group | dose(mg/kg) | number of animals | gastric lesion area(mm²) | lesion inhibition rate(%) |
|---|---|---|---|---|
| control | - | 5 | 65.8±13.05 | - |
| ranitidine | 30 | 7 | 34.8±4.83* | 47.1 |
| rebamipide | 30 | 7 | 40.1±7.43 | 39.1 |
| extract from bark of L. tulipifera | 3 | 7 | 49.5±9.04 | 24.8 |
| | 10 | 7 | 29.3±4.10* | 55.4 |
| | 30 | 7 | 31.2±5.20* | 52.5 |

| | | | | |
|---|---|---|---|---|
| *: Significant difference compared to the control group (p<0.05) | | | | |

Efficacy of extract from bark of Liriodendron tulipifera obtained in Preparation Example 1 according to acid inducing stomach ulcer model has been shown in Table 6. The gastric lesion area of control group has been 65.8±13.05mm². The lesion inhibition rates have been measured into 24.8%, 55.4%, 52.5% respectively with respect to 3mg/kg, 10mg/kg, 30mg/kg administration groups. Therefore, the lesion area has been reduced regarding administration groups in a dose dependent manner.

### (Example 5) Acute toxicity test of rat by extract from bark of Liriodendron tulipifera

Using 7 week old specific pathogen-free SD rats, the acute toxicity test has been carried out regarding extract from bark of Liriodendron tulipifera obtained in Preparation Example 1. Experimental animal has been selected by the appearance of animal. Then, the healthy animal has been employed after more than 7 days breeding. The breeding conditions have been adjusted according to environment controlled rearing system, which are 22±2 °C of temperature, 50±5% of relative humidity, 150∼600Lux of illumination, 12 hours of illumination time (07:00∼19:00). The animals have taken the feed freely in the form of solid feed for experimental animal. Further, the water has been also freely supplied. The composition of feed has been designed by supplier. Of course, there has been no factor influencing the experimental result.

In the experimental group, 10 rats consisting of 5 male rats and 5 female rats have been employed. After dissolving extract from bark of Liriodendron tulipifera in 30% Cremophor EL, the test material has been dissolved in saline solution, the dose of administration into rat has been 2g/kg of extract from bark of Liriodendron tulipifera. After administration of test material, clinical observation of experimental animals has been made for 14 days in a frequency of once per day. After finishing the experiment, gastrointestinal and chest organs of experimental animal have been observed under ether anesthesia. As a result of experiment, there has been no outstanding change of clinical symptom or change of body weight in respect to all animals administered by extract from bark of Liriodendron tulipifera obtained in Preparation Example 1. Of course, there has been no lethal animal. The results have been shown in Tables 7, 8 and 9.

**[Table 7] The mortality rate of rat by orally administering extract from bark of Liriodendron tulipifera**

| | dose (g/kg) | mortality rate | number of lethal animal | | | | | | | | | | | | | |
|---|---|---|---|---|---|---|---|---|---|---|---|---|---|---|---|---|
| | | | days after administration | | | | | | | | | | | | | |
| | | | 1 | 2 | 3 | 4 | 5 | 6 | 7 | 8 | 9 | 10 | 11 | 12 | 13 | 14 |
| control | | 0/10 | 0 | 0 | 0 | 0 | 0 | 0 | 0 | 0 | 0 | 0 | 0 | 0 | 0 | 0 |
| extract from bark of L. tulipifera | 2 | 0/10 | 0 | 0 | 0 | 0 | 0 | 0 | 0 | 0 | 0 | 0 | 0 | 0 | 0 | 0 |

**[Table 8] The change of clinical symptom of rat by orally administering extract from bark of Liriodendron tulipifera**

| | dose (g/kg) | change of clinical symptom | | | | | | | | | | | | | |
|---|---|---|---|---|---|---|---|---|---|---|---|---|---|---|---|
| | | days after administration | | | | | | | | | | | | | |
| | | 1 | 2 | 3 | 4 | 5 | 6 | 7 | 8 | 9 | 10 | 11 | 12 | 13 | 14 |
| control | | 0 | 0 | 0 | 0 | 0 | 0 | 0 | 0 | 0 | 0 | 0 | 0 | 0 | 0 |
| extract from bark of L. tulipifera | 2 | 0 | 0 | 0 | 0 | 0 | 0 | 0 | 0 | 0 | 0 | 0 | 0 | 0 | 0 |

**[Table 9] The change of body weight of rat by orally administering extract from bark of Liriodendron tulipifera**

| | | dose (g/kg) | body weight(g) | | |
|---|---|---|---|---|---|
| | | | days after administration | | |
| | | | 1 | 7 | 14 |
| male | control | | 225.2±2.95 | 284.8±4.66 | 326.8±4.09 |
| | extract from bark of L. tulipifera | 2 | 226.4±1.67 | 291.2±5.36 | 333.0±7.84 |
| female | control | | 156.4±7.70 | 188.0±5.70 | 202.4±6.35 |
| | extract from bark of L. tulipifera | 2 | 152.0±4.42 | 183.6±5.37 | 196.8±7.19 |

## Claims

1. A pharmaceutical composition for preventing or treating gastritis and/or stomach ulcer from the bark of Liriodendron tulipifera comprising 0.01∼50 wt% of epitulipinolide and costunolide as active ingredients.

2. The pharmaceutical composition according to claim 1, further comprising epitulipdienolide, ridentin and/or deacetyllipiferolide as another active ingredient.

3. The pharmaceutical composition according to claim 1, further comprising the pharmaceutically acceptable carrier.

4. The oral preparation comprising the pharmaceutical composition of claim 3, wherein said preparation is at least one selected from of tablet, pill, powder, hard capsule, gelatin banding hard capsule, soft capsule, chewing tablet in the form of caramel or jelly and/or oral solution.

5. The oral preparation according to claim 4, wherein said oral preparation is selected from soft capsule or oral solution.
